# EUROPEAN PATENT APPLICATION

(11) **EP 4 537 871 A1**
(43) Date of publication of application: **16.04.2025**
(21) Application number: 23862699.8
(22) Date of filing: 17.04.2023
(51) Int. Cl.: A61M 5/142, A61M 5/315

(54) **DRUG SOLUTION ADMINISTRATION DEVICE**

(30) Priority: 07.09.2022 JP 2022142253
(71) Applicant: Terumo Kabushiki Kaisha, Tokyo 151-0072 (JP)
(72) Inventor: KONDO, Akira, Ashigarakami-gun, Kanagawa 259-0151 (JP); NAKANISHI, Masaru, Ashigarakami-gun, Kanagawa 259-0151 (JP); HASEGAWA, Makoto, Ashigarakami-gun, Kanagawa 259-0151 (JP)
(74) Representative: KIPA AB
(86) International application number: PCT/JP2023/015285
(87) International publication number: WO 2024/053149

(57) **Abstract**

In a liquid medicine administration device (10), in a case where a protruding end of a second protrusion (110) of a second fitting portion (108) comes into contact with a protruding end of a first protrusion (54) of a first fitting portion (52) in a contact state between the first fitting portion (52) and the second fitting portion (108) and the second fitting portion (108) is not fitted to the first fitting portion (52), a fitting facilitating guide portion (90) guides a guided portion (149) provided on a pusher (84) with rotation of a feed screw shaft (86) when rotation restriction on the pusher (84) by a rotation restricting structure (88) is released such that the pusher (84) rotates while moving in a distal direction to fit the second fitting portion (108) to the first fitting portion (52).

## Description

### Technical Field

The present invention relates to a liquid medicine administration device.

### Background Art

Japanese Patent No. 5653217 discloses a liquid medicine administration device including a vial (liquid medicine container), a stopper (gasket), a pressing mechanism, and a drive portion. The gasket is slidably disposed inside the liquid medicine container. The pressing mechanism includes a pusher for pressing the gasket in a distal direction and a feed screw shaft for moving the pusher in the distal direction. The drive portion rotates the feed screw shaft.

In such a liquid medicine administration device, the rotation of the pusher is restricted by a frictional force generated between an inner peripheral surface of the liquid medicine container and an outer peripheral surface of the gasket, thereby preventing the pusher from rotating together with the feed screw shaft when the feed screw shaft is rotated.

### Summary of Invention

Meanwhile, in order to maintain a liquid medicine in the liquid medicine container in a sterile state in an initial state of the liquid medicine administration device, the pusher may be disposed apart from the gasket in a proximal direction, and the pusher may be moved in the distal direction in a state where the rotation of the pusher is restricted by a rotation restricting structure. In addition, in order to restrict the rotation of the pusher with respect to the gasket, a first fitting portion having an uneven shape may be provided on a distal end surface of the pusher, and a second fitting portion having an uneven shape to be fitted to the first fitting portion may be provided on a proximal end surface of the gasket.

In such a liquid medicine administration device, when the rotation restriction of the pusher by the rotation restricting structure is released before the gasket reaches the most distal end in the liquid medicine container, and the rotation restriction of the pusher is switched from the rotation restricting structure to the frictional force generated between the gasket and the liquid medicine container, it is preferable to lead to downsizing of the liquid medicine administration device.

However, in the liquid medicine administration device, there is a case where a protruding end of a protrusion of the first fitting portion and a protruding end of a protrusion of the second fitting portion come into contact with each other due to dimensional tolerance, assembly tolerance, or the like of components when the pusher is moved in the distal direction and brought into contact with the gasket. In this case, since the first fitting portion is not fitted to the second fitting portion, the pusher rotates (idles) with respect to the gasket when the rotation restriction of the pusher by the rotation restricting structure is released. Therefore, the gasket may not be pressed in the distal direction by the pusher.

An object of the present invention is to solve the above problems.
(1) One aspect of the present invention is a liquid medicine administration device including: a liquid medicine container storing a liquid medicine and having a liquid medicine discharge port at a distal end; a gasket liquid-tightly disposed to be slidable in the liquid medicine container; a pressing mechanism including a pusher that presses the gasket in a distal direction and a feed screw shaft that has a second screw portion, screwed into a first screw portion provided in the pusher, and moves the pusher in the distal direction; and a drive portion that rotates the feed screw shaft, wherein the gasket includes a first fitting portion having an uneven shape on a proximal end surface, the pusher includes a second fitting portion, which has an uneven shape and is fitted to the first fitting portion, on a distal end surface, the second fitting portion is spaced apart from the first fitting portion in a proximal direction in an initial state of the liquid medicine administration device, the pressing mechanism includes a rotation restricting structure formed to restrict rotation of the pusher along a rotation direction of the feed screw shaft to move the pusher in the distal direction and bring the second fitting portion into contact with the first fitting portion, and to release the restriction of the rotation of the pusher before the gasket reaches a most distal end in the liquid medicine container in a state where the second fitting portion is in contact with the first fitting portion, and a fitting facilitating guide portion that guides a guided portion, provided in the pusher with rotation of the feed screw shaft, to rotate the pusher while moving the pusher in the distal direction and fit the second fitting portion to the first fitting portion when the restriction of the rotation of the pusher by the rotation restricting structure is released in a case where a protruding end of a protrusion of the second fitting portion is in contact with a protruding end of a protrusion of the first fitting portion in a contact state between the first fitting portion and the second fitting portion and the second fitting portion is not fitted to the first fitting portion, and the rotation of the pusher with respect to the gasket is restricted and the rotation of the pusher is restricted by a frictional force between the gasket and the liquid medicine container in a fitted state where the first fitting portion and the second fitting portion are fitted to each other.
(2) In the liquid medicine administration device according to item (1), the pusher includes an intermediate plunger having the first screw portion and a third screw portion, and a pressing plunger that has a fourth screw portion screwed into the third screw portion and presses the gasket, a distal end of the second screw portion is located in the proximal direction with respect to a distal end of the feed screw shaft, the intermediate plunger moves in the distal direction with respect to the feed screw shaft by the rotation of the feed screw shaft, and rotates together with the feed screw shaft in a state where the first screw portion is located at the distal end of the second screw portion, and the pressing plunger moves in the distal direction with respect to the intermediate plunger by the rotation of the intermediate plunger.
(3) In the liquid medicine administration device according to item (2), the intermediate plunger is spaced apart from the liquid medicine container.
(4) The liquid medicine administration device according to any one of items (1) to (3), including a support portion that supports the liquid medicine container, wherein the rotation restricting structure includes a first restricting portion provided in the pusher, and a second restricting portion provided on the support portion, and the first restricting portion abuts on the second restricting portion to restrict the rotation of the pusher along the rotation direction of the feed screw shaft.
(5) In the liquid medicine administration device according to item (4), the second restricting portion is located in a direction facing a proximal end surface of the liquid medicine container.
(6) In the liquid medicine administration device according to item (4) or (5), the first restricting portion includes a first engaging portion, the second restricting portion includes a second engaging portion, one of the first engaging portion and the second engaging portion includes a restricting recess, and the other of the first engaging portion and the second engaging portion includes a restricting protrusion fitted into the restricting recess.
(7) In the liquid medicine administration device according to item (6), the second engaging portion extends along an axial direction of the pusher.
(8) In the liquid medicine administration device according to any one of items (4) to (7), the first restricting portion includes an abutting portion facing the rotation direction of the feed screw shaft, and the second restricting portion includes a stopper portion on which the abutting portion abuts.
(9) In the liquid medicine administration device according to item (8), the stopper portion is a flat surface extending along an axial direction of the pusher.
(10) In the liquid medicine administration device according to item (8), the fitting facilitating guide portion includes an inclined surface inclined in a circumferential direction of the pusher from the stopper portion toward the distal direction of the pusher.
(11) In the liquid medicine administration device according to any one of items (4) to (10), the guided portion is provided in the first restricting portion.
(12) In the liquid medicine administration device according to any one of items (4) to (11), the fitting facilitating guide portion is integrally molded with the second restricting portion.
(13) In the liquid medicine administration device according to any one of items (1) to (12), a distal portion of the pusher is located at a proximal portion inside the liquid medicine container in the initial state.

According to the present invention, the pusher is disposed apart from the gasket in the proximal direction in the initial state of the liquid medicine administration device, so that the liquid medicine in the liquid medicine container can be maintained in an aseptic state. When the drive portion rotates the feed screw shaft, rotation of the pusher along the rotation direction of the feed screw shaft is restricted by the rotation restricting structure, so that the pusher moves in the distal direction, and the second fitting portion of the pusher comes into contact with the first fitting portion of the gasket. Then, when the second fitting portion is fitted to the first fitting portion, the rotation restriction of the pusher is switched from the rotation restricting structure to the frictional force generated between the gasket and the liquid medicine container before the gasket reaches the most distal end in the liquid medicine container. As a result, the liquid medicine administration device can be downsized as compared with a case where the rotation of the pusher is restricted by the rotation restricting structure until the gasket reaches the most distal end in the liquid medicine container.

In addition, there is a case where the protruding end of the protrusion of the first fitting portion and the protruding end of the protrusion of the second fitting portion come into contact with each other so that the second fitting portion is not fitted to the first fitting portion depending on dimensional tolerance and assembly tolerance of components of the liquid medicine administration device. Even in such a case, the pusher rotates while moving in the distal direction by the fitting facilitating guide portion when the rotation restriction of the pusher by the rotation restricting structure is released, so that the second fitting portion can be fitted to the first fitting portion. Therefore, the gasket can be pressed in the distal direction by the pusher.

### Brief Description of Drawings

Fig. 1 is a partially omitted schematic plan view of a liquid medicine administration device according to an embodiment of the present invention.
Fig. 2 is a partially omitted cross-sectional view of the liquid medicine administration device in Fig. 1.
Fig. 3 is a perspective view of a gasket in Fig. 2.
Fig. 4 is a partially enlarged cross-sectional view of the liquid medicine administration device in Fig. 2.
Fig. 5 is a transverse cross-sectional view taken along line V-V in Fig. 2.
Fig. 6 is a partially exploded perspective view of a pressing mechanism in Fig. 2.
Fig. 7 is an explanatory cross-sectional view illustrating a state where a drive unit is pushed into a housing such that a connection needle penetrates a sealing member.
Fig. 8 is an explanatory cross-sectional view illustrating a state where a protruding end of a first protrusion of a first fitting portion and a protruding end of a second protrusion of a second fitting portion are in contact with each other.
Fig. 9 is an explanatory cross-sectional view illustrating a state where the second fitting portion is fitted to the first fitting portion.
Fig. 10 is a transverse cross-sectional view taken along line X-X in Fig. 9.
Fig. 11 is a cross-sectional explanatory view illustrating a state where the gasket is pressed to the most distal end in a liquid medicine container.

### Description of Embodiments

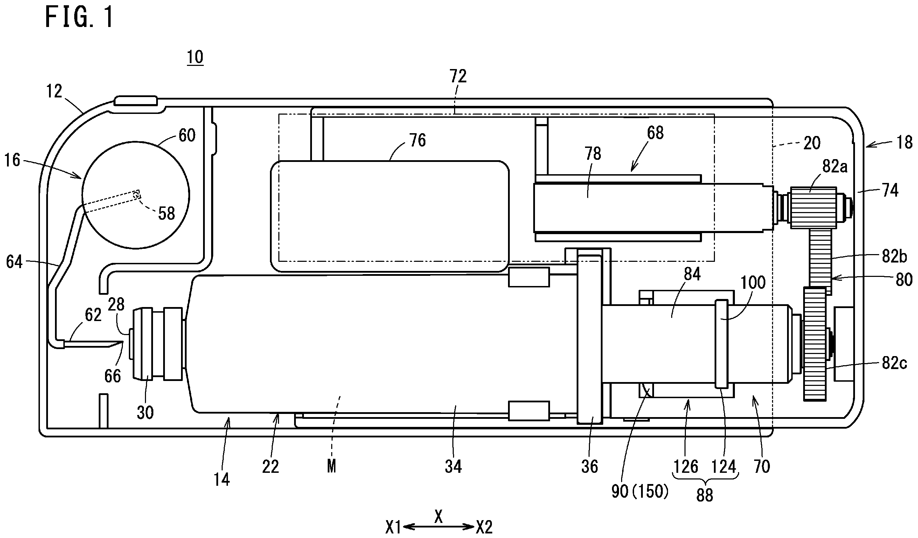

As illustrated in Fig. 1, a liquid medicine administration device 10 according to an embodiment of the present invention is used for administering a liquid medicine M into a living body. The liquid medicine administration device 10 continuously administers the liquid medicine M in a prefilled syringe 14 into the living body over a relatively long time (for example, about several minutes to several hours). The liquid medicine administration device 10 may intermittently administer the liquid medicine M into the living body. Examples of the liquid medicine M include protein preparations, narcotic analgesics, and diuretics.

The liquid medicine administration device 10 includes a housing 12, the prefilled syringe 14, a puncture unit 16, and a drive unit 18.

The housing 12 extends along an axial direction (arrow X direction) of the prefilled syringe 14. The housing 12 is formed, for example, by joining two cover members to each other in a thickness direction of the housing 12 orthogonal to the arrow X direction (direction orthogonal to the paper surface of Fig. 1). Note that Fig. 1 does not illustrate one (upper) cover member of the housing 12.

The housing 12 houses the puncture unit 16, the prefilled syringe 14, and the drive unit 18. The puncture unit 16 is attached to one end (end in an arrow X1 direction) of the housing 12. The other end (end in an arrow X2 direction) of the housing 12 is provided with an opening 20 for inserting the drive unit 18 into the housing 12.

An insertion hole (not illustrated) through which a puncture needle 58 forming the puncture unit 16 is inserted is formed in a wall portion of the housing 12 to which the puncture unit 16 is attached. An adhesive sheet (not illustrated) for attaching the liquid medicine administration device 10 to skin of the living body is fixed to an outer surface of the wall portion.

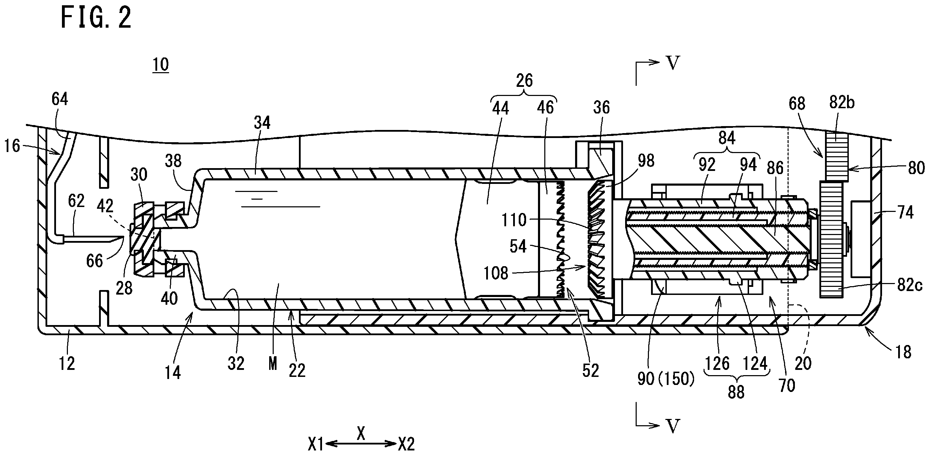

As illustrated in Fig. 2, the prefilled syringe 14 includes a liquid medicine container 22, a liquid medicine M, a gasket 26, a sealing member 28, and a distal end cap 30.

The liquid medicine container 22 stores the liquid medicine M. The liquid medicine container 22 is formed in a hollow cylindrical shape having a liquid medicine chamber 32 therein. The liquid medicine container 22 includes a body portion 34, a flange portion 36, a shoulder portion 38, and a nozzle 40. The body portion 34 extends along the axial direction (arrow X direction) of the prefilled syringe 14. An inner diameter and an outer diameter of the body portion 34 are constant (substantially constant) over an entire length of the body portion 34. A proximal end opening is formed at a proximal end of the body portion 34. The flange portion 36 protrudes radially outward from the proximal end (end in the arrow X2 direction) of the body portion 34 and extends annularly.

A diameter of the shoulder portion 38 decreases from the distal end (end in the arrow X1 direction) of the body portion 34. The nozzle 40 protrudes in a distal direction (arrow X1 direction) from a radially inner end of the shoulder portion 38. The nozzle 40 is formed in a tubular shape. A liquid medicine discharge port 42 communicating with the liquid medicine chamber 32 is formed on a distal end of the nozzle 40. The inside of the liquid medicine container 22 is filled with the liquid medicine M in advance.

The gasket 26 includes a gasket main body 44 and a connection member 46. The gasket main body 44 is slidably disposed inside the liquid medicine container 22. The gasket main body 44 is configured to be made of, for example, an elastic resin material such as a rubber material or an elastomer material. An outer peripheral surface of the gasket main body 44 is in close contact with an inner peripheral surface of the body portion 34 of the liquid medicine container 22 in a liquid-tight manner.

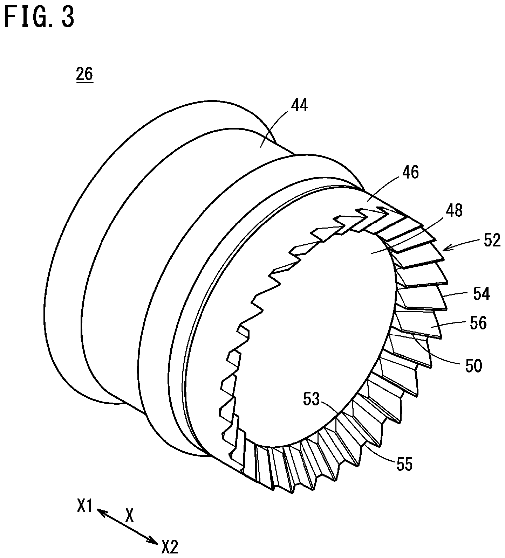

In Fig. 3, the connection member 46 is connected to a proximal portion of the gasket main body 44. The connection member 46 is configured to be made of, for example, a resin material or a metal material harder than the material forming the gasket main body 44. A proximal end surface of the connection member 46 includes a first proximal end surface 48 and a second proximal end surface 50.

The first proximal end surface 48 is located at the center of the proximal end surface of the connection member 46. The first proximal end surface 48 is formed in a circular shape. The first proximal end surface 48 is a flat surface extending in a direction orthogonal to an axis of the connection member 46.

The second proximal end surface 50 is located radially outward of the connection member 46 with respect to the first proximal end surface 48. The second proximal end surface 50 annularly extends so as to surround the first proximal end surface 48. The second proximal end surface 50 is inclined in the arrow X2 direction toward the radially outer side of the connection member 46.

The second proximal end surface 50 is provided with a first fitting portion 52 having an uneven shape. The first fitting portion 52 extends annularly. The first fitting portion 52 has a shape in which first protrusions 54 (first teeth) and first recesses 56 (first tooth grooves) are alternately arranged in a circumferential direction of the connection member 46.

Each of the first protrusions 54 protrudes from the second proximal end surface 50. Each of the first protrusions 54 is formed such that a length (tooth thickness) along the circumferential direction of the connection member 46 decreases in a protruding direction. Each of the first protrusions 54 extends from a boundary 53 between the first proximal end surface 48 and the second proximal end surface 50 to an outer peripheral end 55 of the second proximal end surface 50. The first recess 56 is located between the adjacent first protrusions 54. Bottom surfaces of the first recesses 56 are formed by the second proximal end surface 50.

As illustrated in Fig. 2, the sealing member 28 liquid-tightly seals the liquid medicine discharge port 42 of the nozzle 40. The sealing member 28 is configured to be made of an elastic resin material such as a rubber material or an elastomer material. The distal end cap 30 prevents the sealing member 28 from coming out of the liquid medicine discharge port 42. The distal end cap 30 is configured to be made of, for example, a hard resin material.

In Fig. 1, the puncture unit 16 includes the puncture needle 58, a needle support portion 60, a connection needle 62, and a needle coupling portion 64. The puncture needle 58 has a needle tip capable of puncturing the skin of the living body. The puncture needle 58 is formed in a tubular shape. The puncture needle 58 extends along the thickness direction of the housing 12. The needle support portion 60 is arranged in the housing 12 to support the puncture needle 58. The puncture needle 58 moves in the distal direction of the puncture needle 58 by operating a puncture switch (not illustrated).

The connection needle 62 has a needle tip 66 that can penetrate the sealing member 28. The connection needle 62 is formed in a tubular shape. The connection needle 62 extends along the axial direction (arrow X direction) of the prefilled syringe 14. The needle tip 66 of the connection needle 62 faces the arrow X2 direction. In an initial state of the liquid medicine administration device 10, the needle tip 66 of the connection needle 62 is located apart from the sealing member 28 in the arrow X1 direction.

The needle coupling portion 64 couples a proximal end of the connection needle 62 and a proximal end of the puncture needle 58 to each other. The needle coupling portion 64 is a flexible tube that communicates a lumen of the connection needle 62 and a lumen of the puncture needle 58 with each other.

The drive unit 18 presses the gasket 26 in the distal direction. The drive unit 18 is slidably inserted into the housing 12 together with the prefilled syringe 14 in the distal direction (arrow X1 direction) of the prefilled syringe 14.

The drive unit 18 includes a drive portion 68, a pressing mechanism 70, a controller 72, and a drive cover 74. The drive portion 68 includes a power supply unit 76, a motor 78, and a power transmission mechanism 80. The power supply unit 76 supplies power to the motor 78 and the controller 72. The power supply unit 76 is located on the lateral side of the liquid medicine container 22. The power supply unit 76 is located in the arrow X2 direction of the needle support portion 60.

The power supply unit 76 includes, for example, a plurality of batteries disposed in series in the arrow X direction. The batteries may be either primary batteries or secondary batteries. The power supply unit 76 may include one battery or the plurality of batteries as described above. The motor 78 is located in the arrow X2 direction of the power supply unit 76. The power transmission mechanism 80 transmits a rotational driving force of the motor 78 to the pressing mechanism 70. The power transmission mechanism 80 includes, for example, a drive gear 82a, an intermediate gear 82b, and a driven gear 82c. The power transmission mechanism 80 may adopt an appropriate configuration.

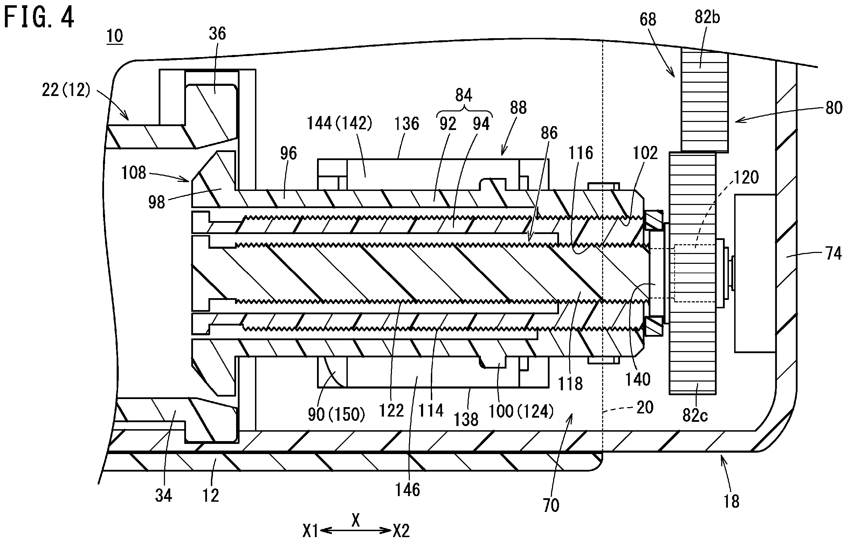

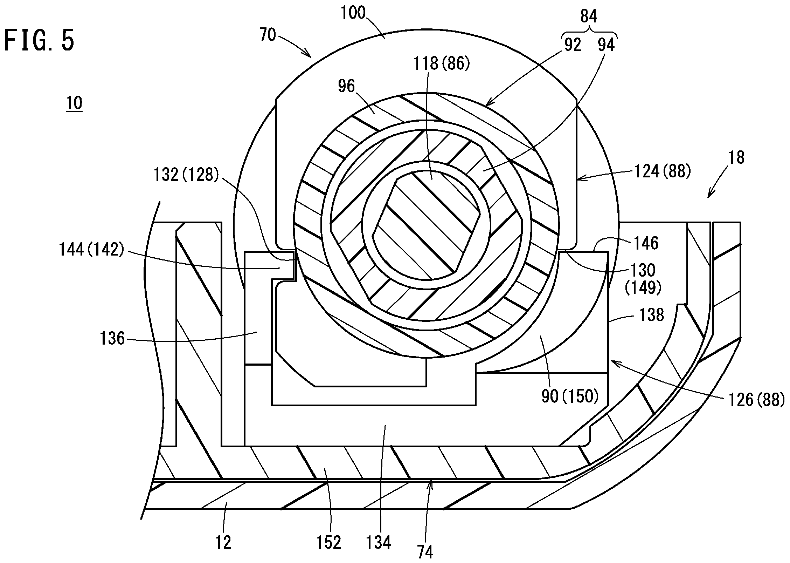

As illustrated in Figs. 4 and 5, the pressing mechanism 70 includes a pusher 84, a feed screw shaft 86, a rotation restricting structure 88, and a fitting facilitating guide portion 90. In the initial state of the liquid medicine administration device 10, a distal portion of the pusher 84 is located at a proximal portion inside the liquid medicine container 22. The pusher 84 includes a pressing plunger 92 having a tubular shape and an intermediate plunger 94 disposed inside the pressing plunger 92. That is, the pusher 84 has a telescopic structure that can extend in the axial direction.

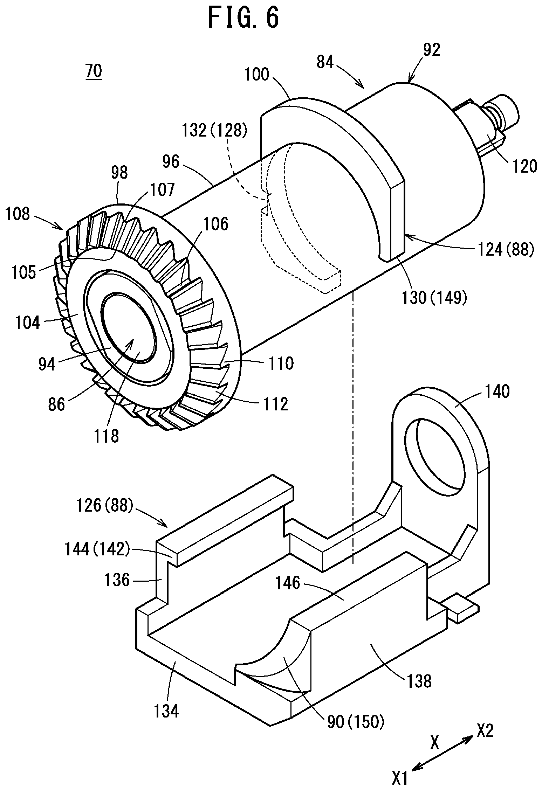

In Fig. 6, the pressing plunger 92 is integrally molded using, for example, a resin material. However, the pressing plunger 92 may be configured to be made of a metal material. The pressing plunger 92 includes a plunger main body 96, a plunger distal portion 98, and an outward protruding portion 100. The plunger main body 96 is formed in a cylindrical shape. A female screw 102 is formed at a proximal portion of an inner peripheral surface of the plunger main body 96 (see Fig. 4).

The plunger distal portion 98 is connected to a distal end of the plunger main body 96. An outer diameter of the plunger distal portion 98 is larger than an outer diameter of the plunger main body 96 and smaller than the inner diameter of the body portion 34 of the liquid medicine container 22. The plunger distal portion 98 supports the distal portion of the pressing plunger 92 in a predetermined posture inside the liquid medicine container 22. In other words, the plunger distal portion 98 suppresses excessive inclination of an axis of the pressing plunger 92 with respect to an axis of the liquid medicine container 22 inside the liquid medicine container 22.

A distal end surface of the pressing plunger 92 includes a first distal end surface 104 and a second distal end surface 106. The first distal end surface 104 is located at the center of the distal end surface of the pressing plunger 92 and extends in an annular shape. The first distal end surface 104 is a flat surface extending in a direction orthogonal to the axis of the pressing plunger 92.

The second distal end surface 106 is located radially outward of the pressing plunger 92 with respect to the first distal end surface 104. The second distal end surface 106 extends annularly so as to surround the first distal end surface 104. The second distal end surface 106 is inclined in the arrow X2 direction toward the radially outer side of the pressing plunger 92.

The second distal end surface 106 is provided with a second fitting portion 108 having an uneven shape to be fitted to the first fitting portion 52. The second fitting portion 108 extends annularly. The second fitting portion 108 has a shape in which second protrusions 110 (second teeth) and second recesses 112 (second tooth grooves) are alternately arranged in a circumferential direction of the pressing plunger 92.

Each of the second protrusions 110 protrudes from the second distal end surface 106. Each of the second protrusions 110 is formed such that a length (tooth thickness) along a circumferential direction of the plunger distal portion 98 decreases in a protruding direction. Each of the second protrusions 110 extends from a boundary 105 between the first distal end surface 104 and the second distal end surface 106 to ah outer peripheral end 107 of the second distal end surface 106. The second recess 112 is located between the adjacent second protrusions 110. Bottom surfaces of the second recesses 112 are formed by the second distal end surface 106.

The second protrusion 110 and the second recess 112 are formed so as to be fittable to the first recess 56 and the first protrusion 54, respectively. Rotation of the pressing plunger 92 (pusher 84) with respect to the connection member 46 (gasket 26) is restricted in a state where the second fitting portion 108 is fitted to the first fitting portion 52. Shapes, sizes, numbers, arrangements, and the like of the first protrusions 54, the first recesses 56, the second protrusions 110, and the second recesses 112 can be appropriately set.

The outward protruding portion 100 protrudes radially outward from an intermediate portion of an outer peripheral surface of the plunger main body 96. The outward protruding portion 100 is a plate-like portion extending in a circumferential direction of the plunger main body 96. The outward protruding portion 100 supports an intermediate portion of the pressing plunger 92 in a predetermined posture inside the liquid medicine container 22. In other words, the outward protruding portion 100 prevents the axis of the pressing plunger 92 from being excessively inclined with respect to the axis of the liquid medicine container 22 inside the liquid medicine container 22.

The intermediate plunger 94 is formed in a cylindrical shape. In the initial state of the liquid medicine administration device 10, a distal end of the intermediate plunger 94 does not protrude in the distal direction from a distal end of the pressing plunger 92. The intermediate plunger 94 is spaced apart from the liquid medicine container 22.

As illustrated in Fig. 4, a male screw 114 screwed into the female screw 102 of the pressing plunger 92 is formed on an outer peripheral surface of the intermediate plunger 94. The male screw 114 is not formed at a distal portion of the outer peripheral surface of the intermediate plunger 94. In other words, a distal end of the male screw 114 is located in a proximal direction (arrow X2 direction) with respect to the distal end of the intermediate plunger 94. Therefore, the pressing plunger 92 does not come out of the intermediate plunger 94 in the distal direction. Note that a proximal end of the male screw 114 is located at a proximal end of the intermediate plunger 94. A female screw 116 is formed at a proximal portion of an inner peripheral surface of the intermediate plunger 94.

The feed screw shaft 86 includes a rod portion 118 and a coupling portion 120. In the initial state of the liquid medicine administration device 10, a distal end of the rod portion 118 does not protrude in the distal direction from the distal end of the pressing plunger 92.
A male screw 122 screwed into the female screw 116 of the intermediate plunger 94 is formed on an outer peripheral surface of the rod portion 118.

The male screw 122 is not formed at a distal portion of the outer peripheral surface of the rod portion 118. In other words, a distal end of the male screw 122 is located in the proximal direction (arrow X2 direction) with respect to a distal end of the feed screw shaft 86. Therefore, the intermediate plunger 94 does not come out of the rod portion 118 in the distal direction. Note that a proximal end of the male screw 122 is located at a proximal end of the rod portion 118. The coupling portion 120 is connected to the proximal end of the rod portion 118. The driven gear 82c of the drive portion 68 is coupled to the coupling portion 120.

Such a pressing mechanism 70 includes the female screw 116 (first screw portion) of the intermediate plunger 94, the male screw 122 (second screw portion) of the feed screw shaft 86, the male screw 114 (third screw portion) of the intermediate plunger 94, and the female screw 102 (fourth screw portion) of the pressing plunger 92. In the pressing mechanism 70, frictional resistance between the female screw 102 of the pressing plunger 92 and the male screw 114 of the intermediate plunger 94 is larger than frictional resistance between the male screw 122 of the feed screw shaft 86 and the female screw 116 of the intermediate plunger 94.

As illustrated in Figs. 4 to 6, the rotation restricting structure 88 restricts rotation of the pusher 84 along a rotation direction of the feed screw shaft 86. The rotation restricting structure 88 is formed such that the rotation restriction of the pusher 84 is released before the gasket 26 reaches the most distal end in the liquid medicine container 22 in a state where the second fitting portion 108 is in contact with the first fitting portion 52. The rotation restricting structure 88 includes a first restricting portion 124 and a second restricting portion 126. The first restricting portion 124 is provided on the outward protruding portion 100 of the pusher 84.

In Fig. 6, the first restricting portion 124 includes a first engaging portion 128 and an abutting portion 130. The first engaging portion 128 includes a restricting recess 132 formed in the outward protruding portion 100. The restricting recess 132 penetrates the outward protruding portion 100 in the axial direction of the pusher 84. The restricting recess 132 is opened radially outward of the pusher 84.

The abutting portion 130 faces the rotation direction of the feed screw shaft 86. The abutting portion 130 is a flat surface. The abutting portion 130 is at a position rotated by about 180° in the circumferential direction of the plunger main body 96 from the restricting recess 132.

The second restricting portion 126 is attached to a support portion 152 (to be described later) of the drive cover 74 (see Fig. 5). The second restricting portion 126 includes a base portion 134, a first rib 136, and a second rib 138. The base portion 134 is a plate-like portion disposed on the support portion 152 (see Fig. 5). The base portion 134 extends along the axial direction of the pusher 84. The base portion 134 is provided with a bearing 140 that rotatably supports the coupling portion 120 of the feed screw shaft 86.

In Fig. 5, the first rib 136 and the second rib 138 protrude in a direction opposite to the support portion 152 from both side portions of the base portion 134 in a width direction. The pusher 84 (plunger main body 96) is located between the first rib 136 and the second rib 138. As illustrated in Fig. 6, the first rib 136 and the second rib 138 extend in parallel to each other along the axial direction of the pusher 84.

The first rib 136 is provided with a second engaging portion 142 that engages with the first engaging portion 128. The second engaging portion 142 includes a restricting protrusion 144 protruding from a protruding end portion of the first rib 136 toward the second rib 138. In the initial state of the liquid medicine administration device 10, the restricting protrusion 144 is fitted into the restricting recess 132 (see Fig. 5). The restricting protrusion 144 extends along the axial direction (arrow X direction) of the pusher 84.

A protruding end surface of the second rib 138 is provided with a stopper portion 146 on which the abutting portion 130 abuts. The stopper portion 146 is a flat surface extending along the axial direction of the pusher 84. A length of the stopper portion 146 along the axial direction of the pusher 84 is substantially the same as a length of the restricting protrusion 144 along the axial direction of the pusher 84. The rotation restricting structure 88 is formed such that the restricting recess 132 is separated from the restricting protrusion 144 when the abutting portion 130 is separated from the stopper portion 146 in a case where the pusher 84 (pressing plunger 92) is moved in the distal direction with respect to the feed screw shaft 86.

When a protruding end of the second protrusion 110 comes into contact with a protruding end of the first protrusion 54 and the second fitting portion 108 is not fitted to the first fitting portion 52, the fitting facilitating guide portion 90 guides a guided portion 149 provided in the pusher 84 with the rotation of the feed screw shaft 86 such that the pusher 84 rotates while moving in the distal direction to fit the second fitting portion 108 to the first fitting portion 52. In the present embodiment, the guided portion 149 is the abutting portion 130 of the first restricting portion 124.

The fitting facilitating guide portion 90 is integrally molded with the second restricting portion 126. The fitting facilitating guide portion 90 includes an inclined surface 150 continuous to the stopper portion 146.

The inclined surface 150 is inclined in a circumferential direction of the pusher 84 (the circumferential direction of the pressing plunger 92) toward the distal direction of the pusher 84. The inclined surface 150 terminates at a position rotated by about 90° in the circumferential direction of the pusher 84 from a boundary portion with the stopper portion 146 (see Fig. 5). The inclined surface 150 is a flat surface.

In Fig. 1, the controller 72 controls an operation of the motor 78. The drive cover 74 integrally supports the power supply unit 76, the motor 78, the power transmission mechanism 80, the pusher 84, and the controller 72. The drive cover 74 includes the support portion 152 that supports the liquid medicine container 22 of the prefilled syringe 14 (see Fig. 5).

Next, a method for using the liquid medicine administration device 10 will be described. As illustrated in Fig. 2, in the initial state of the liquid medicine administration device 10, the second fitting portion 108 is located apart from the first fitting portion 52 in the proximal direction (arrow X2 direction). In addition, the sealing member 28 of the prefilled syringe 14 is located apart from the needle tip 66 of the connection needle 62 in the arrow X2 direction.

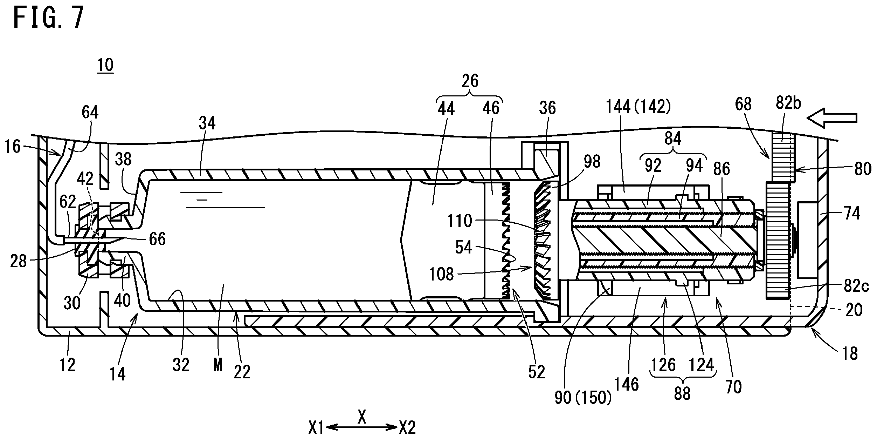

When the liquid medicine administration device 10 is used, as illustrated in Fig. 7, a user pushes the drive unit 18 in the arrow X1 direction with respect to the housing 12 to cause the connection needle 62 to penetrate the sealing member 28 of the prefilled syringe 14. As a result, the lumen of the puncture needle 58 (see Fig. 1) and the liquid medicine chamber 32 communicate with each other via the connection needle 62 and the needle coupling portion 64.

In the liquid medicine administration device 10, the controller 72 drives the motor 78 to administer the liquid medicine M. When the motor 78 is driven, the rotational driving force of the motor 78 is transmitted to the feed screw shaft 86 via the power transmission mechanism 80 (the drive gear 82a, the intermediate gear 82b, and the driven gear 82c), and the feed screw shaft 86 rotates, so that torque acts on the pusher 84.

At this time, since the restricting protrusion 144 is fitted to the restricting recess 132 and the abutting portion 130 abuts on the stopper portion 146, the rotation of the pressing plunger 92 along the rotation direction of the feed screw shaft 86 is restricted. Therefore, the pusher 84 (the pressing plunger 92 and the intermediate plunger 94) linearly moves in the distal direction with respect to the feed screw shaft 86. Then, the second fitting portion 108 of the pusher 84 comes into contact with the first fitting portion 52 of the gasket 26.

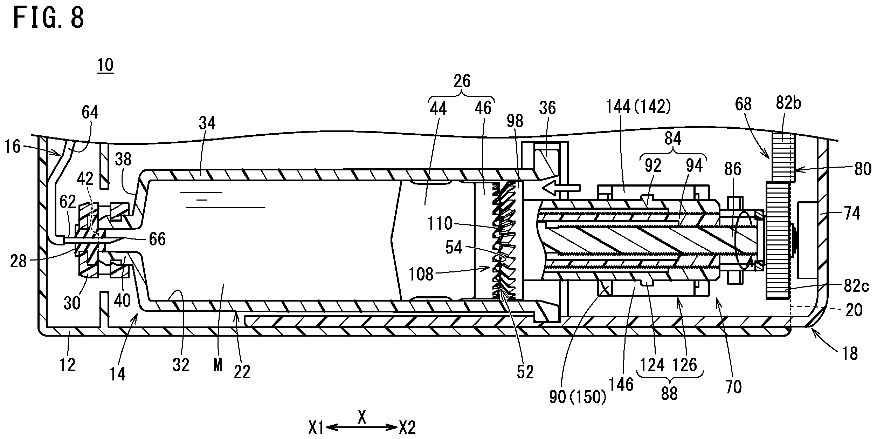

At this time, there is a case where the protruding end of the first protrusion 54 of the first fitting portion 52 and the protruding end of the second protrusion 110 of the second fitting portion 108 come into contact with each other so that the second fitting portion 108 is not fitted to the first fitting portion 52 depending on the dimensional tolerance and the assembly tolerance of the components of the liquid medicine administration device 10 as illustrated in Fig. 8. In such a case, the pusher 84 whose rotation is restricted by the rotation restricting structure 88 presses the gasket 26 in the distal direction (arrow X1 direction) while the protruding end of the first protrusion 54 is in contact with the protruding end of the second protrusion 110. As a result, the gasket 26 moves inside the liquid medicine container 22 in the distal direction, and the liquid medicine M in the liquid medicine container 22 is administered into the living body via the puncture unit 16.

Then, when the restricting recess 132 is detached (separated) from the restricting protrusion 144 and the abutting portion 130 is separated from the stopper portion 146, the abutting portion 130 (guided portion 149) comes into contact with the inclined surface 150.

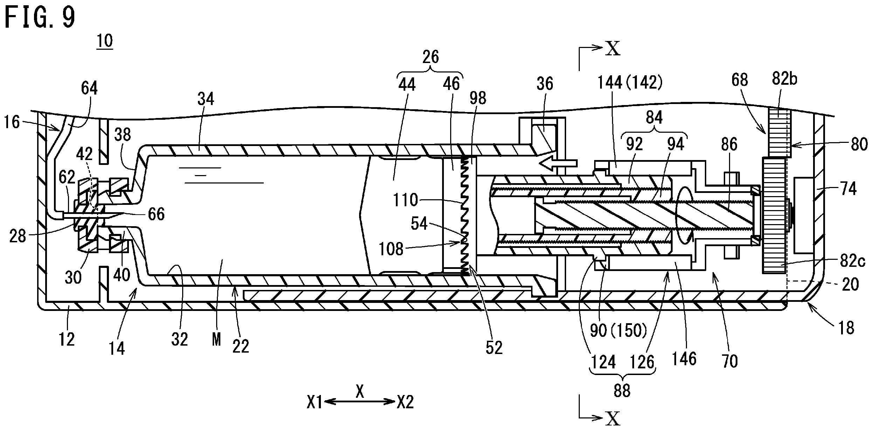

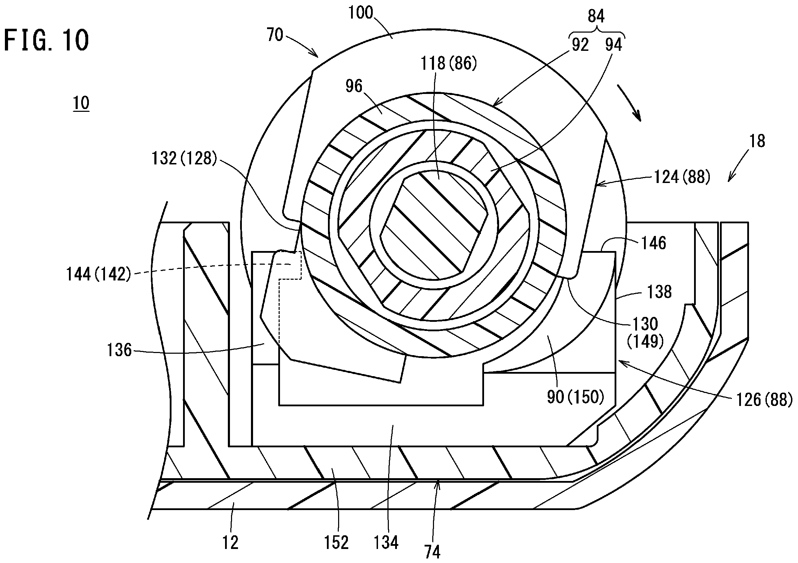

Then, when the feed screw shaft 86 further rotates, the abutting portion 130 moves while making contact with the inclined surface 150 as illustrated in Figs. 9 and 10, so that the pusher 84 (pressing plunger 92) moves in the distal direction (arrow X1 direction) with respect to the feed screw shaft 86 while rotating in the rotation direction of the feed screw shaft 86. As a result, the second fitting portion 108 is fitted to the first fitting portion 52 (the second protrusion 110 is fitted into the first recess 56, and the first protrusion 54 is fitted into the second recess 112).

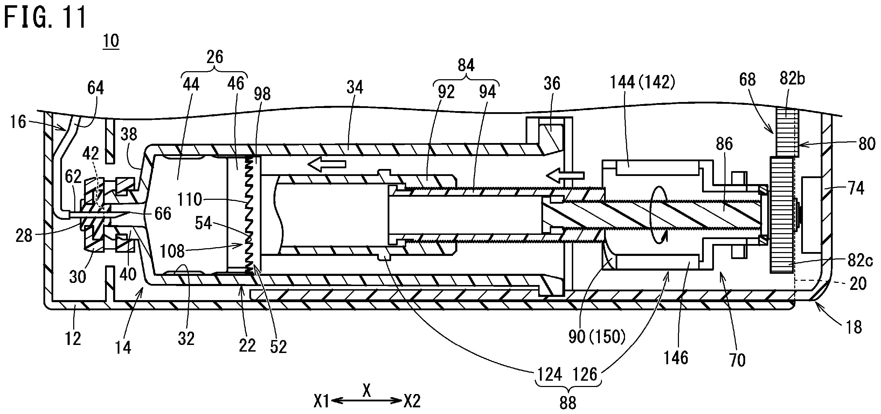

When the second fitting portion 108 is fitted to the first fitting portion 52, the rotation of the pusher 84 along the rotation direction of the feed screw shaft 86 is restricted by a frictional force generated between the outer peripheral surface of the gasket main body 44 and the inner peripheral surface of the body portion 34 of the liquid medicine container 22. Therefore, the pusher 84 linearly moves in the distal direction (arrow X1 direction) with the rotation of the feed screw shaft 86. Note that, when the second fitting portion 108 is fitted to the first fitting portion 52, the pusher 84 does not rotate with respect to the feed screw shaft 86, so that the abutting portion 130 is spaced apart from the inclined surface 150. As illustrated in Fig. 11, the gasket 26 moves to the most distal end in the liquid medicine container 22, whereby all the liquid medicine M in the liquid medicine container 22 is administered into the living body.

In the above description, the case where the protruding end of the second protrusion 110 comes into contact with the protruding end of the first protrusion 54 and the second fitting portion 108 is not fitted to the first fitting portion 52 when the pusher 84 of the liquid medicine administration device 10 in the initial state moves in the distal direction and the second fitting portion 108 comes into contact with the first fitting portion 52 has been exemplified. In this case, the second fitting portion 108 is fitted to the first fitting portion 52 by the fitting facilitating guide portion 90 as described above.

On the other hand, in the present embodiment, there is also a case where the first fitting portion 52 is fitted to the second fitting portion 108 when the pusher 84 of the liquid medicine administration device 10 in the initial state moves in the distal direction and the first fitting portion 52 comes into contact with the second fitting portion 108 depending on the dimensional tolerance and the assembly tolerance of the components of the liquid medicine administration device 10. In such a case, when the second fitting portion 108 is fitted to the first fitting portion 52, the frictional force generated between the outer peripheral surface of the gasket main body 44 and the inner peripheral surface of the body portion 34 of the liquid medicine container 22 restricts the rotation of the pusher 84 along the rotation direction of the feed screw shaft 86. Therefore, when the restricting recess 132 is detached from the restricting protrusion 144 and the abutting portion 130 is separated from the stopper portion 146, the pusher 84 moves in the distal direction without being rotated by the feed screw shaft 86. That is, since the abutting portion 130 does not come into contact with the inclined surface 150, the function of the fitting facilitating guide portion 90 is not exerted.

The present embodiment has the following effects.

According to the present embodiment, the pusher 84 is disposed apart from the gasket 26 in the proximal direction in the initial state of the liquid medicine administration device 10, so that the liquid medicine M in the liquid medicine container 22 can be maintained in an aseptic state. When the drive portion 68 rotates the feed screw shaft 86, the rotation of the pusher 84 along the rotation direction of the feed screw shaft 86 is restricted by the rotation restricting structure 88, so that the pusher 84 moves in the distal direction, and the second fitting portion 108 of the pusher 84 comes into contact with the first fitting portion 52 of the gasket 26. Then, when the second fitting portion 108 is fitted to the first fitting portion 52, the rotation restriction of the pusher 84 is switched from the rotation restricting structure 88 to the frictional force generated between the gasket 26 and the liquid medicine container 22 before the gasket 26 reaches the most distal end in the liquid medicine container 22. As a result, the liquid medicine administration device 10 can be downsized as compared with a case where the rotation of the pusher 84 is restricted by the rotation restricting structure 88 until the gasket 26 reaches the most distal end in the liquid medicine container 22.

In addition, there is a case where the protruding end of the first protrusion 54 of the first fitting portion 52 and the protruding end of the second protrusion 110 of the second fitting portion 108 come into contact with each other so that the second fitting portion 108 is not fitted to the first fitting portion 52 depending on the dimensional tolerance and the assembly tolerance of the components of the liquid medicine administration device 10. Even in such a case, the pusher 84 rotates while moving in the distal direction by the fitting facilitating guide portion 90 when the rotation restriction of the pusher 84 by the rotation restricting structure 88 is released, so that the second fitting portion 108 can be fitted to the first fitting portion 52. Therefore, the gasket 26 can be pressed in the distal direction by the pusher 84.

The pusher 84 includes the intermediate plunger 94 and the pressing plunger 92. The intermediate plunger 94 has the female screw 116 (first screw portion) and the male screw 114 (third screw portion). The pressing plunger 92 has the female screw 102 (fourth screw portion) screwed into the male screw 114 and presses the gasket 26. The distal end of the male screw 122 (second screw portion) of the feed screw shaft 86 is located in the proximal direction with respect to the distal end of the feed screw shaft 86. The intermediate plunger 94 moves in the distal direction with respect to the feed screw shaft 86 by the rotation of the feed screw shaft 86, and rotates together with the feed screw shaft 86 in a state where the female screw 116 of the intermediate plunger 94 is located at the distal end of the male screw 122 of the feed screw shaft 86. The pressing plunger 92 moves in the distal direction with respect to the intermediate plunger 94 by the rotation of the intermediate plunger 94.

According to such a configuration, since the pusher 84 can extend, a length of the liquid medicine administration device 10 along the axial direction of the liquid medicine container 22 can be made short. As a result, the liquid medicine administration device 10 can be further downsized.

The intermediate plunger 94 is spaced apart from the liquid medicine container 22.

According to such a configuration, since no frictional force is generated between the liquid medicine container 22 and the intermediate plunger 94, a driving force required to move the gasket 26 in the distal direction can be efficiently reduced.

The liquid medicine administration device 10 includes the support portion 152 that supports the liquid medicine container 22. The rotation restricting structure 88 includes the first restricting portion 124 provided in the pusher 84 and the second restricting portion 126 provided in the support portion 152. The first restricting portion 124 abuts on the second restricting portion 126 to restrict the rotation of the pusher 84 along the rotation direction of the feed screw shaft 86.

According to such a configuration, it is possible to restrict the rotation of the pusher 84 along the rotation direction of the feed screw shaft 86 with a simple configuration.

The second restricting portion 126 is located in a direction facing a proximal end surface of the liquid medicine container 22.

According to such a configuration, the rotation restricting structure 88 can be downsized.

The first restricting portion 124 has the first engaging portion 128, and the second restricting portion 126 has the second engaging portion 142. The first engaging portion 128 includes the restricting recess 132. The second engaging portion 142 includes the restricting protrusion 144 fitted to the restricting recess 132.

According to such a configuration, the rotation of the pusher 84 along the rotation direction of the feed screw shaft 86 can be efficiently restricted by the restricting recess 132 and the restricting protrusion 144.

The second engaging portion 142 extends along the axial direction of the pusher 84.

According to such a configuration, the pusher 84 can be moved in the distal direction in a state where the rotation of the pusher 84 is restricted with a simple configuration.

The first restricting portion 124 has the abutting portion 130 facing the rotational direction of the feed screw shaft 86. The second restricting portion 126 has the stopper portion 146 on which the abutting portion 130 abuts.

According to such a configuration, it is possible to restrict the rotation of the pusher 84 along the rotation direction of the feed screw shaft 86 with a simple configuration.

The stopper portion 146 is a flat surface extending along the axial direction of the pusher 84.

According to such a configuration, the pusher 84 can be moved in the distal direction in a state where the rotation of the pusher 84 is restricted with a simple configuration. In addition, the torque acting on the pusher 84 (abutting portion 130) can be received by the flat surface.

The guided portion 149 is provided in the first restricting portion 124.

According to such a configuration, it is not necessary to provide the guided portion 149 in the pusher 84 separately from the first restricting portion 124 so that the configuration of the pusher 84 can be simplified.

The fitting facilitating guide portion 90 is integrally molded with the second restricting portion 126.

According to such a configuration, rigidity of the fitting facilitating guide portion 90 and the second restricting portion 126 can be enhanced.

The fitting facilitating guide portion 90 includes the inclined surface 150 inclined in the circumferential direction of the pusher 84 from the stopper portion 146 toward the distal direction of the pusher 84.

According to such a configuration, the first restricting portion 124 can be smoothly shifted from the stopper portion 146 to the inclined surface 150.

In the initial state of the liquid medicine administration device 10, a distal portion of the pusher 84 is located at a proximal portion inside the liquid medicine container 22.

According to such a configuration, it is possible to increase the amount of the liquid medicine M that can be stored in the liquid medicine container 22 while suppressing an increase in size of the liquid medicine administration device 10.

The present embodiment is not limited to the above-described configuration. The first engaging portion 128 may have a restricting protrusion, and the second engaging portion 142 may have a restricting recess. The rotation restricting structure 88 may include only the abutting portion 130 and the stopper portion 146 without including the first engaging portion 128 and the second engaging portion 142. In addition, the rotation restricting structure 88 may include only the first engaging portion 128 and the second engaging portion 142 without including the abutting portion 130 and the stopper portion 146. The first fitting portion 52 and the second fitting portion 108 do not necessarily have a gear shape. The shapes, positions, sizes, numbers, and the like of the first fitting portion 52 and the second fitting portion 108 can be appropriately set as long as the first fitting portion 52 and the second fitting portion 108 can be fitted to each other to restrict the rotation of the pressing plunger 92 with respect to the gasket 26.

Note that the present invention is not limited to the above disclosure, and various configurations can be adopted without departing from the gist of the present invention.

## Claims

1. A liquid medicine administration device comprising:
a liquid medicine container storing a liquid medicine and having a liquid medicine discharge port at a distal end;
a gasket liquid-tightly disposed to be slidable in the liquid medicine container;
a pressing mechanism comprising a pusher that presses the gasket in a distal direction and a feed screw shaft that has a second screw portion, screwed into a first screw portion provided in the pusher, and moves the pusher in the distal direction; and
a drive portion that rotates the feed screw shaft,
wherein the gasket comprises a first fitting portion having an uneven shape on a proximal end surface,
the pusher comprises a second fitting portion, which has an uneven shape and is fitted to the first fitting portion, on a distal end surface,
the second fitting portion is spaced apart from the first fitting portion in a proximal direction in an initial state of the liquid medicine administration device,
the pressing mechanism comprises
a rotation restricting structure formed to restrict rotation of the pusher along a rotation direction of the feed screw shaft to move the pusher in the distal direction and bring the second fitting portion into contact with the first fitting portion, and to release the restriction of the rotation of the pusher before the gasket reaches a most distal end in the liquid medicine container in a state where the second fitting portion is in contact with the first fitting portion, and
a fitting facilitating guide portion that guides a guided portion provided in the pusher with rotation of the feed screw shaft in such a manner that the pusher rotates while moving in the distal direction to fit the second fitting portion to the first fitting portion when the restriction of the rotation of the pusher by the rotation restricting structure is released in a case where a protruding end of a protrusion of the second fitting portion is in contact with a protruding end of a protrusion of the first fitting portion in a contact state between the first fitting portion and the second fitting portion and the second fitting portion is not fitted to the first fitting portion, and
the rotation of the pusher with respect to the gasket is restricted and the rotation of the pusher is restricted by a frictional force between the gasket and the liquid medicine container in a fitted state where the first fitting portion and the second fitting portion are fitted to each other.

2. The liquid medicine administration device according to claim 1, wherein
the pusher comprises
an intermediate plunger having the first screw portion and a third screw portion, and
a pressing plunger that has a fourth screw portion screwed into the third screw portion and presses the gasket,
a distal end of the second screw portion is located in the proximal direction with respect to a distal end of the feed screw shaft,
the intermediate plunger moves in the distal direction with respect to the feed screw shaft by the rotation of the feed screw shaft, and rotates together with the feed screw shaft in a state where the first screw portion is located at the distal end of the second screw portion, and
the pressing plunger moves in the distal direction with respect to the intermediate plunger by the rotation of the intermediate plunger.

3. The liquid medicine administration device according to claim 2, wherein
the intermediate plunger is spaced apart from the liquid medicine container.

4. The liquid medicine administration device according to claim 1, comprising a support portion that supports the liquid medicine container,
wherein the rotation restricting structure comprises
a first restricting portion provided in the pusher, and
a second restricting portion provided on the support portion, and
the first restricting portion abuts on the second restricting portion to restrict the rotation of the pusher along the rotation direction of the feed screw shaft.

5. The liquid medicine administration device according to claim 4, wherein
the second restricting portion is located in a direction facing a proximal end surface of the liquid medicine container.

6. The liquid medicine administration device according to claim 4, wherein
the first restricting portion comprises a first engaging portion,
the second restricting portion comprises a second engaging portion,
one of the first engaging portion and the second engaging portion comprises a restricting recess, and
another of the first engaging portion and the second engaging portion comprises a restricting protrusion fitted into the restricting recess.

7. The liquid medicine administration device according to claim 6, wherein
the second engaging portion extends along an axial direction of the pusher.

8. The liquid medicine administration device according to claim 4, wherein
the first restricting portion comprises an abutting portion facing the rotation direction of the feed screw shaft, and
the second restricting portion comprises a stopper portion on which the abutting portion abuts.

9. The liquid medicine administration device according to claim 8, wherein
the stopper portion is a flat surface extending along an axial direction of the pusher.

10. The liquid medicine administration device according to claim 8, wherein
the fitting facilitating guide portion comprises an inclined surface inclined in a circumferential direction of the pusher from the stopper portion toward the distal direction of the pusher.

11. The liquid medicine administration device according to claim 4, wherein
the guided portion is provided in the first restricting portion.

12. The liquid medicine administration device according to claim 4, wherein
the fitting facilitating guide portion is integrally molded with the second restricting portion.

13. The liquid medicine administration device according to any one of claims 1 to 12, wherein
in the initial state, a distal portion of the pusher is located at a proximal portion inside the liquid medicine container.
